# EUROPEAN PATENT APPLICATION

(11) **EP 3 939 699 A1**
(43) Date of publication of application: **19.01.2022**
(21) Application number: 20186490.7
(22) Date of filing: 17.07.2020
(51) Int. Cl.: B01L 3/00, C12M 3/06, C12M 1/42, C12M 1/00

(54) **GROWTH MODULATION**

(71) Applicant: Biomillenia SAS, 93230 Romainville (FR)
(72) Inventor: DAJKOVIC, Aleksander, 75018 Paris (FR); DU, Guansheng, 94200 Ivry sur Seine (FR); LOEFFERT, Dirk, 42781 Hann (FR)
(74) Representative: CH Kilger Anwaltspartnerschaft mbB

(57) **Abstract**

The present invention uses microfluidics droplets for studying the growth dynamics of communities of bacteria in the presence of various perturbations such as various chemicals or other microorganisms and to establish microbial assemblies model which is representative of a microbiota of interest and to examine their growth dynamics after perturbations such as with an array of chemicals or other microorganisms. In particular, the present invention refers to a method and apparatus for assessing the effect of a chemical or biological substance on the growth of microorganisms.

## Description

### Field of the invention

The present invention is in the field of microbiology and relates to methods for measuring the modulation of bacterial growth. In particular, the present invention relates to the use of droplet microfluidics to study the effects of chemicals or other microorganisms on the growth dynamics of assemblies of microorganisms.

### Background of the invention

Solid media for cultivation of microorganisms have been used since early 19th century and remain the golden standard for microbiologists. They allow the isolation of single colonies of bacteria and yeasts. The addition of antibiotics or precise formulation of media composition, such that only a subset of microorganisms are able to grow, can render the growth media selective. One major drawback of cultivation of microorganisms on Petri plates containing solid media is the very low throughput. Some efforts have been made to automate their use of mainly to improve throughput for diagnostics (Previ Isola, Biomerieux) or screening (Colony Piking robot, Tecan). Both solutions rely on robotics, which are expensive, complicated to put in place and run, require a significant investment and are not practical for studying interactions between different organisms or between microorganisms and arrays of chemicals.

Another classical approach involves the use of liquid cultures in various containers: classically tubes and flasks, and more recently microtiter plates. Microtiter plates allow parallelization and the possibility of working with larger numbers of samples. They remain the standard solution for parallelization, high-throughput screening, handling of chemical or mutant libraries as well as for antibiotic susceptibility testing. Automatizing microtiter plates was developed by various companies specialized in robotics and is used to increase the throughput for screening in industrial settings and implement more complex workflows. It remains limited by the heterogeneities inherent in the microtiter-based culture (multiple interfaces, poor mixing, evaporation of small volumes, etc). Also, measuring growth modulation of a biological system such as bacterial cultures commonly requires a high number of replicates to compensate for the inherent variability in growth kinetics. Hence, even using robotic solutions remains expensive, technologically complicated to put in place, and limited in throughput.

Some effort has been made to develop time lapse microscopy coupled with microchannels where growth of individual cells can be dynamically followed using video microscopy. Said method is powerful and can be used to study fine growth dynamics and influences of various compounds. Nevertheless, this method is limited in throughput and by the kind of interactions that can be studied. Only the influence of selected chemicals can be examined and not the effects of other microbes.

In fundamental microbiology and in various industrial applications of microbiology there is an interest in measuring the modulation of bacterial growth of either individual species or of a comprehensive population of all bacteria making up a specific ecological niche, the microbiota. Microbiota is the sum of all microorganisms populating a given ecological niche such as different (human) skin areas, the gut, the oral cavity, but also different environments such as soil, water. With the advent of the research on the microbiome, it has become clear that besides pathogenic bacteria that are harmful to living organisms, there are many bacterial species that interact in positive ways to stimulate homeostasis. Because of this expanding impact on microbiology on all aspects of human and environmental health, there is increasing interest in understanding the dynamics of various microbial communities and how they change in response to perturbations. It is of interest to understand the conditions that stimulate the growth and development of microbial species with beneficial and harmful effects. Due to the complexities of microbial communities that constitute most of the microbiota of interest (human, animal, plant, and environmental), standard microbiological methods, such as screening for growth modulation by optical density measurements in microtiter plates, do not provide the necessary throughput to capture growth dynamics of a large number of strains under a variety of conditions (presence of various chemicals, other strains, etc). The use of microfluidic technology surmounts this problem of throughput by using droplets as incubation vessels for microbes and increasing the throughput by orders of magnitude. The present invention reports the use of droplet microfluidics for studying the growth dynamics of communities of bacteria in the presence of various perturbations such as various chemicals or other microorganisms. The present invention can be used to establish model microbial assemblies representative of microbiota of interest (skin microbiota, intestinal, plant, etc) and to examine their growth dynamics after perturbations such as with an array of chemicals or other microorganisms.

### Brief description of the invention

The present invention uses microfluidics droplets for studying the growth dynamics of communities of bacteria in the presence of various perturbations such as various chemicals or other microorganisms and to establish microbial assemblies model which is representative of a microbiota of interest and to examine their growth dynamics after perturbations such as with an array of chemicals or other microorganisms.

These microbiotas models are then used for testing for interactions with arrays of chemical compounds or with other microbial species as follows:
- High-throughput incorporation of a chemical library (or a microorganism or microorganisms of interest whose effect is to be tested) in microfluidic droplets along with the appropriate barcodes for facile downstream identification of active extracts
- Encapsulation of single cells of each individual bacterial strain from the model microbiota in microfluidic droplets along with appropriate barcodes
- Fusion of droplets containing the chemical library (or a microorganism of interest) with the droplets containing bacteria
- Incubation under appropriate conditions (temperature and atmosphere, i.e. aerobic and anaerobic)
- Sorting of droplets based on the effects of the chemical compounds on growth (some chemicals may enhance the growth of microorganisms and lead to the production of higher biomass, some chemicals may have neutral effects in they produce no change in the microbial biomass, and some chemicals may have inhibitory effects where the production of biomass by the microorganisms is detectably diminished)
- Identification of active compounds by analysis of the barcodes and downstream bioinformatic analysis

### Detailed description of the invention

The present invention relates to the use of droplet microfluidics to study the effects of chemicals or other microorganisms on the growth dynamics of assemblies of microorganisms.

As used herein, the term "microorganism" refers to bacteria, fungi, yeast, archaea, viruses and phages.

As used herein, the term "biological substance" refers to plant extracts, supernatants of bacterial or yeast cultures, antibodies, peptides, carbohydrate molecules, lipids, proteins, etc.

As used herein, the term "chemical substance" refers to any material with a definite chemical composition and comprises any organic or inorganic substance of a particular molecular identity and may exist in different physical states such as a liquid, a solid or gas. Examples comprise but are not limiting to alcohols, aldehydes, esters, terpenes, aromatics, ketones, lactones, thiols, hormones, amines, siderophores, acids, antimicrobials, fungicides, toxins.

As used herein, the term "chemical or biological library" refers to the collection of biological or chemical substances organized in an ordered way that may contain hundreds or thousands of different substances

As used herein, the term "microbiota" refers to ecological communities of commensal, symbiotic and pathogenic microorganisms found in and on all multicellular organisms studied to date from plants to animals. Microbiota includes bacteria, archaea, protists, fungi and viruses.

As used herein, the term "growth medium" refers to a solid, liquid or semi-solid designed to support the growth of microorganisms or cells. Different types of media are used for growing the different types of cells.

As used herein, a "microfluidic system" is a "microfluidic device" or "microfluidic chip" or "synthesis chip" or "lab-on-a-chip" or "chip" is a unit or device that permits the manipulation and transfer of microliters or nanoliters or picoliters of liquid ("droplet", "microfluidic droplet") into a substrate comprising micro-channels. The device is configured to allow the manipulation of liquids, including reagents and solvents, to be transferred or conveyed within the micro channels and reaction chamber using mechanical or non-mechanical pumps.

As used herein, the term "droplet" refers to a measure of volume and further relates to an isolated portion of a fluid. As used herein, the terms "first", "second" and "third" population of droplets are used to discriminate droplets according to their content. As the method is performed in a microfluidic system, the term "droplet" also refers to "microfluidic droplet".

Microbiota are of great importance for human health and all earth ecosystems, yet current research is hampered by the absence of standardized and reproducible model microbial systems. The adoption of standard model organisms in cell and molecular biology allowed great leaps in the understanding of physiological processes by enabling reproducible experimentation and controlled perturbations. Microbiome research and various associated industrial applications would likewise benefit from the establishment of carefully designed model communities of microbes that could be used for controlled experimentation as a representative of the more complex microbiota.

On the industrial side, e.g. the cosmetics and pharmaceutical industries are interested in testing the effects of various chemical compounds on the human microbiota. The development of a microbial community model with sufficient complexity to accurately represent these microbiomes would greatly facilitate such testing. Microfluidic technology permits the creation of such a microbial community system as well as the subsequent testing of the effects of various chemical compounds and/or other microorganisms on the microbiome.

To create a microbial community model that accurately represents a microbiota ecosystem, the species of bacteria to include in the model system need to be chosen and isolated from the original microbiota in pure culture. They are then stored in microtiter plates and revived for the need of experiments. Using the power of microfluidic technology, the microbiota model is hardy limited to specific number of species but commonly include from approximately up to 100 species to up to 1000 species. These microbiotas models are then used for testing for interactions with arrays of chemical compounds or with other microbial species as follows:
- High-throughput incorporation of a chemical library (or a microorganism or microorganisms whose effect is to be tested) in microfluidic droplets along with the appropriate barcodes for facile downstream identification of active extracts
- Encapsulation of single cells of each individual bacterial strain from the model microbiota in microfluidic droplets along with appropriate barcodes
- Fusion of droplets containing the chemical library (or a microorganism of interest) with the droplets containing bacteria
- Incubation under appropriate conditions (temperatures in the range of 10C to 95C and under aerobic, microaerophilic or anaerobic atmosphere conditions)
- Sorting of droplets on based the effects of the chemical compounds on growth (enhancement, neutrality, or inhibition)
- Identification of active compounds by analysis of the barcodes and downstream bioinformatic analysis

According to the present invention, barcodes can be of different nature such as but not limited to barcodes consisting of nucleotide (DNA) combinations that can be analyzed by sequencing or varying concentrations of dyes or mixtures of dyes, more preferably of fluorescent dyes or fluorescent particles. The current invention is not thought to limit the investigation of microbiota to those inhabiting living organisms such as humans, animals or plants. Although the current invention describes bacterial species, the microbiota also comprises fungi, yeast, archaea, viruses and phages and shall not be limited to bacteria only.

The present invention refers to a method for assessing the effect of a chemical or biological substance on the growth of microorganisms comprising the steps of:
a) generating a first population of droplets, wherein said droplets of first population comprise chemical or biological compounds from a chemical or biological library
b) generating a second population of droplets, wherein said second population of droplets comprise microorganisms
c) fusing the droplets of first and second population, thereby generating a third population of droplets, wherein said third population comprises compounds from a chemical or biological library and microorganisms;
d) incubating said third population of droplets under specific conditions of temperature and atmosphere; (conditions)
e) analyzing the effect of the chemical or biological compounds on the growth of microorganisms; the effects can include enhancement or inhibtion of microbial biomass production

In one embodiment, the chemical or biological substance is selected from the group comprising an antibody, an organic, an inorganic compound, a pharmaceutically active substance and/or an organism.

In one embodiment the droplet size is in the 20 pL range.

In one embodiment, the first populations of droplets further comprises a unique DNA barcode.

In one embodiment, the barcodes can be analyzed by sequencing or varying concentrations of dyes or mixtures of dyes, more preferably of fluorescent dyes or fluorescent particles.

In one embodiment, in the droplet library, each individual droplet contains a single chemical compound from the chemical library along with a unique DNA barcode for facile downstream identification by sequencing.

In one embodiment, the microorganism to be analyzed is selected from the group comprising bacteria, fungi and/or viruses.

In one embodiment, the microorganism to be analyzed is an anaerobic bacterium.

In one embodiment, the effect of the chemical or biological compound is analyzed via a potential change of the microbial biomass.

In one embodiment, the effect of the chemical or biological compound is enhancement, neutrality, or inhibition on cell growth.

The method for assessing the effect of a chemical or biological substance on the growth of microrganisms further comprises the step of adding a fluorescent marker (such as a cell viability dye, or a fluorescent dye that specifically stains DNA, RNA, lipids, or peptidoglycan) to the microorganism to be analyzed.

The present invention refers to an microfluidic apparatus for assessing the effect of a chemical or biological substance on the growth of a microorganism comprising:
(a) a first microfluidic device for incorporating a chemical or biological substance into a first population of droplet;
(b) a second microfluidic device for incorporating a microorganism into a second population of droplets;
(c) a microchannel wherein the first and second population of droplets from the first and second microfluidic devices are combined and said first and second population of droplets are fused;
(d) a sorting unit

In one embodiment, the apparatus further comprising a fluorescent detector.

In one embodiment, the sorting unit is connected to a FACS.

In one embodiment, the chemical or biological substance is selected from the group comprising an antibody, an organic, an inorganic compound, a pharmaceutically active substance and/or an organism.

In one embodiment, the droplet size is in the 20 pL range.

In one embodiment, the first populations of droplets further comprises a unique DNA barcode.

In one embodiment, the barcodes can be analyzed by sequencing or varying concentrations of dyes or mixtures of dyes, more preferably of fluorescent dyes or fluorescent particles.

In one embodiment, in the droplet library, each individual droplet contains a single chemical compound from the chemical library along with a unique DNA barcode for facile downstream identification.

In one embodiment, the microorganism to be analyzed is selected from the group comprising bacteria, fungi and/or viruses.

In one embodiment, the microorganism to be analyzed is an anaerobic bacterium.

In one embodiment, the effect of the chemical or biological compound is analyzed via fluorescent, OD, or image analysis.

In one embodiment, the effect of the chemical or biological compound is enhancement, neutrality, or inhibition on cell growth.

### High-throughput incorporation of the chemical library in microfluidic droplets

As used herein, a droplet library refers to a library containing a defined chemical array is generated to screen for effects on growth on bacteria. In the droplet library, each individual droplet contains a single chemical compound from the chemical library along with a unique DNA barcode for facile downstream identification. As used herein, the chemical array comprises at least two chemicals and up to thousands of distinct chemicals.

### Encapsulation of representative (skin) bacteria in droplets and incubation.

The encapsulation of bacteria in the appropriate growth medium in microfluidic droplets is performed using specialized microfluidic chips. Briefly, the sample preparation is sufficiently diluted and the bacteria are encapsulated in droplets on a PDMS-based microfluidic chip. Dilution is required to ensure the single bacterial cell per droplet. Droplets are comprised of cells and the culture medium along with the fluorescent dyes (such as a cell viability dye, or a fluorescent dye that specifically stains DNA, RNA, lipids, or peptidoglycan) that are incorporated at the same time or added later, according to the needs of the workflow. Each droplet contains single bacteria of the model microbiome along with a unique barcode. The droplet size is generally in the ^{∼} 20 pL range.

### Fusion of droplets containing bacteria with droplets containing the chemical array and their incubation under appropriate conditions

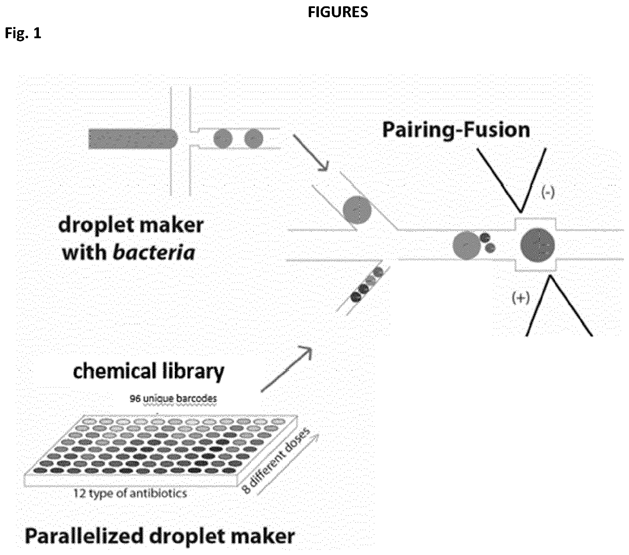

To carry out the testing of the effects of the chemical compounds on the growth of bacteria, the droplets containing the chemical library have to be fused with the droplets containing bacteria. This will be done using the proprietary workflow using a specific microfluidic chip on the Biomillenia platform. The droplets will then be incubated off the chip under the appropriate conditions (temperature and atmosphere) before being reinjected onto a microfluidic chip subjected to sorting. Alternatively, the droplets can also be sorted by other means such as FACS. A schematic representation of this part of the workflow is shown in Figure 1.

### Droplets sorting based on inhibition, enhancement or no effect of bacterial growth and identification of the active compounds.

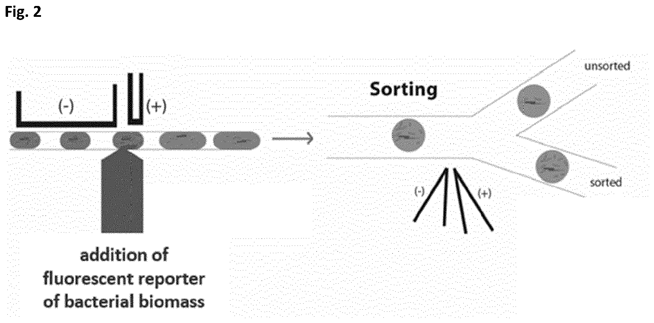

After sufficient incubation of droplets that depends on the growth kinetics of the microbiota or bacterial community under study, but is in the range of 12h-7 days, are subjected to high-throughput sorting on the microfluidic platform, and the droplets containing compounds having the desired activity on growth (enhancement, neutrality, inhibition) are separated from the droplets with no activity (as shown diagrammatically in Figure 2).

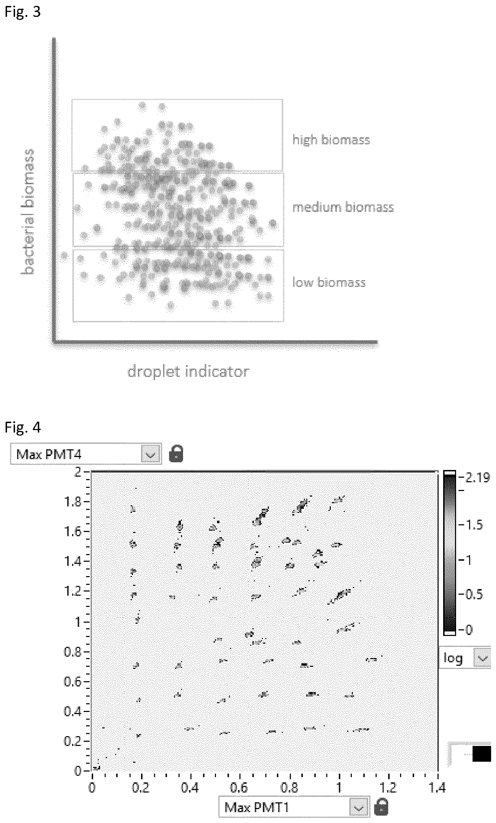

The separation of the droplets based on the effect on growth (enhancement, neutrality, or inhibition) is done by examining the bacterial biomass present in the droplets (as shown diagrammatically below). The bacterial biomass can be detected by proxies for optical density such as but not limited to imaging or fluorescent measurement (e.g. cell staining fluorescent dyes). The gating for sorting is adjusted to include the appropriate fractions of the total bacterial population (Figure 3).

This process can be run either once or will be iterated several times to eliminate possible false-positive and false-negatives by averaging a high number of replicate droplets for each bacterial species. This is easily done by droplet microfluidics but is hardly feasible with any other method such as robotic handling of microtiter plates due to the high number of replicates needed. Bacterial populations of different biomass may be sorted due to variabilities in growth induced by the chemical or biological substances.

The examination (via the barcodes) of the content of the gates from the sample treated by libraries of biological or chemical substances reveals the distributions of species preset. Shifts in these distributions induced by presence of chemicals are indicative of the effect of that specific chemical compound. Given the large number of droplets involved, this statistical approach smooths some of the inevitable biological variability that arises in the system.

Additionally, the affected bacterial species can be identified by color-coding the droplet population with mixtures of varying concentrations of at least 2 fluorescent indicator dyes creating a matrix of droplet population with each a specific dye mixture for each bacterial strain. An example is shown in the Figure 4.

Identification of active and inactive compounds by sequencing of the barcodes and downstream bioinformatic analysis

The active and inactive compounds in these droplets are identified based on the barcodes present. The sorted droplets are demulsified and the DNA contents analyzed for the frequency of the barcodes in the sorted droplet population.

In another application, the microbial model population might not be challenged with regard to the impact of chemical substances or drug compounds on the microbiota model but with the challenge of other bacterial species such as but not limited to pathobiont bacteria.

Typical applications include but are not limited to: a) screening of chemical compound libraries on the growth of the microbiota or specific species thereof such as for cosmetic ingredient screening, pharmaceutical compound screening, environmental pollutions etc.; b) screening of different concentrations of chemical compound libraries, of pharmaceutical compounds, environmental pollutions etc.; c) screening of nutrient factors, d) screening of commensal and or pathobiont strains on growth of the microbiota, and other applications that can be described by analogy. Compound libraries might include but are not limited to chemically synthesized molecules, molecules synthesized by biological means (in vivo production or in vitro making e.g. by cell free systems), molecules extracted from natural sources by appropriate means etc. Compound libraries might be dissolved in various solvents such as aqueous solutions or organic solvents. Depending on the nature of the solvent specific emulsion techniques for droplet making might be needed such as but not limited to surfactants or nanoparticles.

### Brief description of the figures

Figure 1: A schematic representation of encapsulation of bacteria in droplets, fusion of said droplets with droplets containing the chemical array and their incubation under specific conditions.
Figure 2: A schematic representation of sorting droplets based on inhibition, enhancement or no effect of bacterial growth and identification of the active compounds.
Figure 3: Analysis of the bacterial biomass present in the droplets, wherein the biomass can be detected by proxies for optical density.
Figure 4: Bacterial species identified by color-coding the droplet population with mixtures of different concentrations of at least 2 fluorescent indicator dyes by creating a matrix of droplet population, wherein each droplet population for each bacterial strain is labeled with a specific dye mixture.

## Claims

1. A method for assessing the effect of a chemical or biological substance on the growth of microorganisms comprising the steps of:
a) generating a first population of droplets, wherein said droplets of first population comprise chemical or biological compounds from a chemical or biological library
b) generating a second population of droplets, wherein said second population of droplets comprise microorganisms
c) fusing the droplets of first and second population, thereby generating a third population of droplets, wherein said third population comprises compounds from a chemical or biological library and microorganisms;
d) incubating said third population of droplets under specific conditions of temperature and atmosphere; temperatures in the range of 10C to 95C and under aerobic, microaerophilic or anaerobic atmosphere conditions analyzing the effect of the chemical or biological compounds on the growth of microorganisms; the effects can include enhancement or inhibition of microbial biomass.

2. The method according to claim 1, wherein the chemical or biological substance is selected from the group comprising an antibody, an organic, an inorganic compound, a pharmaceutically active substance and/or an organism.

3. The method according to claims 1 to 2, wherein the first droplet further comprises a unique DNA barcode.

4. The method according to claims 1 to 3, wherein the organism to be analysed is selected from the group comprising bacteria, fungi and/or viruses.

5. The method of claims 1 to 4, wherein the organism to be analysed is an anaerobic bacterium.

6. The method of claims 1 to 5, further comprising the step of adding a fluorescent marker to the organism to be analysed.

7. A microfluidic apparatus for assessing the effect of a chemical or biological substance on the growth of a microorganism comprising:
(e) a first microfluidic device for incorporating a chemical or biological substance into a droplet;
(f) a second microfluidic device for incorporating an organism into a population of droplets;
(g) a microchannel wherein the droplets from the first and second microfluidic devices are combined;
(h) a sorting unit

8. The apparatus according to claim 7, further comprising a fluorescent detector.

9. The apparatus according to claims 7 or 8, wherein the sorting unit is a FACS.
